# EUROPEAN PATENT SPECIFICATION

(11) **EP 3 324 054 B1**
(45) Date of publication and mention of the grant of the patent: **20.11.2019**
(21) Application number: 16824284.0
(22) Date of filing: 30.06.2016
(51) Int. Cl.: A61F 2/50, F15B 15/10

(54) **HYDRAULIC ACTUATOR**
HYDRAULISCHER AKTUATOR
ACTIONNEUR HYDRAULIQUE

(30) Priority: 14.07.2015 JP 2015140306; 17.07.2015 JP 2015143028; 17.07.2015 JP 2015143155
(43) Date of publication of application: 23.05.2018
(73) Proprietor: Bridgestone Corporation, Tokyo 104-8340 (JP)
(72) Inventor: OONO, Shingo, Tokyo 104-8340 (JP); SAKURAI, Ryo, Tokyo 104-8340 (JP); SATO, Takayuki, Tokyo 104-8340 (JP)
(74) Representative: Oxley, Robin John George
(86) International application number: PCT/JP2016/069478
(87) International publication number: WO 2017/010304

(56) References cited:
- EP-A1- 0 162 539
- EP-A1- 1 950 424
- JP-A- S6 275 113
- JP-A- H08 170 603
- JP-A- S61 127 905
- US-A- 5 067 390
- US-A- 5 158 005
- US-A- 6 067 892

## Description

### [Technical Field]

The present invention relates to a hydraulic actuator which expands and contracts a tube by using gas or fluid, particularly, a so-called McKibben type hydraulic actuator.

### [Background Art]

Conventionally, in the hydraulic actuator which expands and contracts the tube as described above, a structure (so-called McKibben type structure) including a rubber tube (tubular body) which is expanded and contracted by air pressure, and a sleeve (braided reinforcing structure) which covers an outer periphery of the tube is widely adopted (for example, Patent Literature 1).

Both ends of an actuator main portion formed by the tube and the sleeve are caulked by a sealing member formed of metal.

The sleeve is formed as a tubular structural body in which a high tension fiber such as a polyamide fiber or a metal cord is braided. The sleeve is formed to restrict an expansion movement of the tube within a predetermined region.

Such a hydraulic actuator is used in various fields, especially preferably used as an artificial muscle in nursing equipment or healthcare equipment.

### [Citation List]

### [Patent Literature]

[PTL 1] Japanese Unexamined Patent Application Publication No. S61-236905.

Hydraulic actuators of this type are also already known from the document EP 0162539.

### [Summary of Invention]

In the hydraulic actuator described above, higher contraction force is required. For example, the higher contraction force is required in the hydraulic actuator used in a robot, other than the artificial muscle in the nursing equipment or the healthcare equipment.

In order to obtain such high contraction force, it is considered to adopt oil pressure driving using mineral oil as the fluid. However, in the oil pressure driving, high durability is required because extremely high pressure is applied to the tube and the sleeve.

When the high pressure is applied to an inside of the hydraulic actuator, the actuator main portion might be damaged. Specifically, the sleeve which restricts the expansion region of the tube might be dropped off from the sealing member, or the tube might be damaged due to stress concentration to a specific portion of the tube.

Accordingly, an object of the present invention is, in consideration of the problem described above, to provide a hydraulic actuator having sufficient durability in a case in which high pressure is applied such as a case in which oil pressure driving is adopted.

A hydraulic actuator according to one aspect of the present invention includes an actuator main portion formed by a tube having a cylindrical shape which is expanded and contracted by pressure of fluid and a sleeve formed as a structural body in which cords oriented in a predetermined direction are braided, the sleeve being formed to cover an outer periphery of the tube, and a sealing mechanism which seals an end portion of the actuator main portion in an axial direction.

The sealing mechanism includes a sealing member having a body portion and a flange portion, a caulking member which caulk the actuator main portion in conjunction with the sealing member, and a first locking ring which locks the sleeve. The body portion is inserted into the tube The flange portion is continued to the body portion, and an outer diameter of the flange portion along a radial direction of the actuator main portion is larger than an outer diameter of the body portion. The first locking ring locks the sleeve at an outer side of the body portion in the radial direction. The sleeve has a first folded portion folded via the first locking ring. The caulking member caulk the body portion inserted into the tube, the sleeve located at the outer side of the tube in the radial direction, and the first folded portion in conjunction with the sealing member.

### [Brief Description of Drawings]

[Fig. 1] Fig. 1 is a side view of a hydraulic actuator 10.
[Fig. 2] Fig. 2 is an exploded perspective view of a part of the hydraulic actuator 10.
[Fig. 3] Fig. 3 is a cross-sectional view of a part of the hydraulic actuator 10 along an axial direction D_{AX} of the hydraulic actuator 10 including a sealing mechanism 200 according to an example 1-1.
[Fig. 4] Fig. 4 is a cross-sectional view of a part of the hydraulic actuator 10 along the axial direction D_{AX} of the hydraulic actuator 10 including the sealing mechanism 200 according to an example 1-2.
[Fig. 5] Fig. 5 is a cross-sectional view of a part of the hydraulic actuator 10 along the axial direction D_{AX} of the hydraulic actuator 10 including the sealing mechanism 200 according to an example 1-3.
[Fig. 6] Fig. 6 is a cross-sectional view of a part of the hydraulic actuator 10 along the axial direction D_{AX} of the hydraulic actuator 10 including a sealing mechanism 200A according to an example 2-1.
[Fig. 7] Fig. 7 is a cross-sectional view of a part of the hydraulic actuator 10 along the axial direction D_{AX} of the hydraulic actuator 10 including the sealing mechanism 200A according to an example 2-2.
[Fig. 8] Fig. 8 is a cross-sectional view of a part of the hydraulic actuator 10 along the axial direction D_{AX} of the hydraulic actuator 10 including the sealing mechanism 200A according to an example 2-3.
[Fig. 9] Fig. 9 is a cross-sectional view of a part of the hydraulic actuator 10 along the axial direction D_{AX} of the hydraulic actuator 10 including a sealing mechanism 200B according to an example 3-1.
[Fig. 10] Fig. 10 is a cross-sectional view along the axial direction D_{AX} of the hydraulic actuator 10 including a sealing mechanism 200C according to an example 3-2.
[Fig. 11] Fig. 11 is a developed view of a part of an actuator main portion 100.
[Fig. 12] Fig. 12 is a cross-sectional view along a radial direction of a cord 121 according to a second embodiment.
[Fig. 13] Fig. 13 is a cross-sectional view along a radial direction of a cord 121 according to a third embodiment.

### [Description of Embodiments]

Next, embodiments will be described with reference to drawings. Further, the same or a similar reference numeral is assigned to the same or similar function or configuration, and the description thereof is therefore omitted as needed.

### First embodiment

### (1) Overall schematic configuration of hydraulic actuator

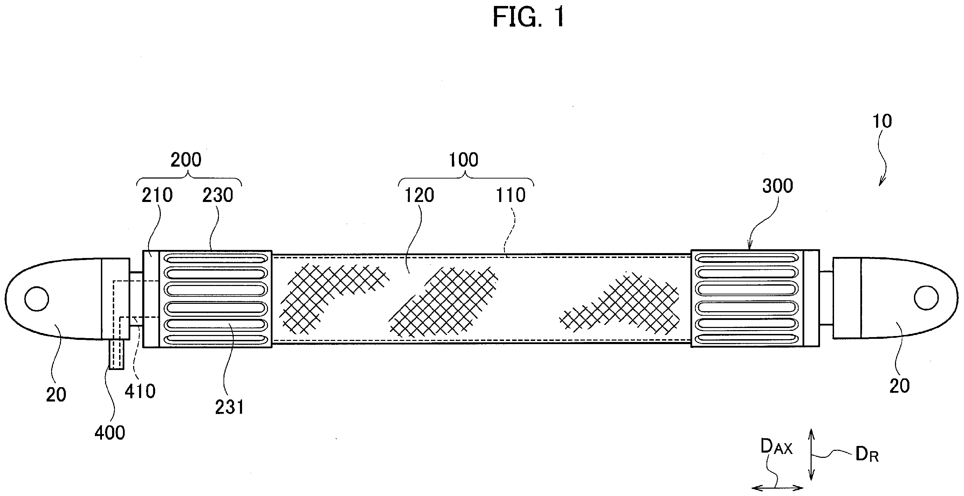

Fig. 1 is a side view of a hydraulic actuator 10 according to the present embodiment. As shown in Fig. 1, the hydraulic actuator 10 is provided with an actuator main portion 100, a sealing mechanism 200, and a sealing mechanism 300. Further, connection portions 20 are formed at both ends of the hydraulic actuator 10, respectively.

The actuator main portion 100 is formed by a tube 110 and a sleeve 120. Fluid flows into the actuator main portion 100 via a fitting 400 and a passing hole 410.

When the fluid flows into the tube 110, the actuator main portion 100 is contracted in an axial direction D_{AX} of the actuator main portion 100 and is expanded in a radial direction D_{R}. Further, when the fluid flows out from the tube 110, the actuator main portion 100 is expanded in the axial direction D_{AX} of the actuator main portion 100 and is contracted in the radial direction D_{R}. With such a shape change of the actuator main portion 100, the hydraulic actuator 10 works as an actuator.

Examples of the fluid used for driving the hydraulic actuator 10 include gas such as air, liquid such as water and mineral oil. Especially, the hydraulic actuator 10 has high durability which can endure oil pressure driving in which high pressure applied to the actuator main portion 100.

Further, such a hydraulic actuator 10 is formed as a so-called McKibben type actuator and is preferably applied to not only an artificial muscle but also a body limb (an upper limb, a lower limb or the like) of a robot in which higher capability (contraction force) is required. A member which forms the body limb or the like is connected to the connection portion 20.

The sealing mechanism 200 and the sealing mechanism 300 are formed to seal both end portions of the actuator main portion 100 in the axial direction D_{AX}. Specifically, the sealing mechanism 200 includes a sealing member 210 and a caulking member 230. The sealing member 210 is formed to seal an end portion of the actuator main portion 100 in the axial direction D_{AX}. Further, the caulking member 230 is formed to caulk the actuator main portion 100 in conjunction with the sealing member 210. A pressed mark 231 is formed on an outer periphery of the crimping member 230 when the caulking member 230 is crimped by a jig.

The difference between the sealing mechanism 200 and the sealing mechanism 300 is whether the fitting 400 (and the passing hole 410) is formed.

The fitting 400 is protruded such that the driving pressure source of the hydraulic actuator 10, specifically a hose (a pipe) connected to a compressor for gas or liquid, is mounted to the fitting 400. The fluid which flows in through the fitting 400 is flows into an inside of the actuator main portion 100, specifically an inside of the tube 110, after passing the passing hole 410.

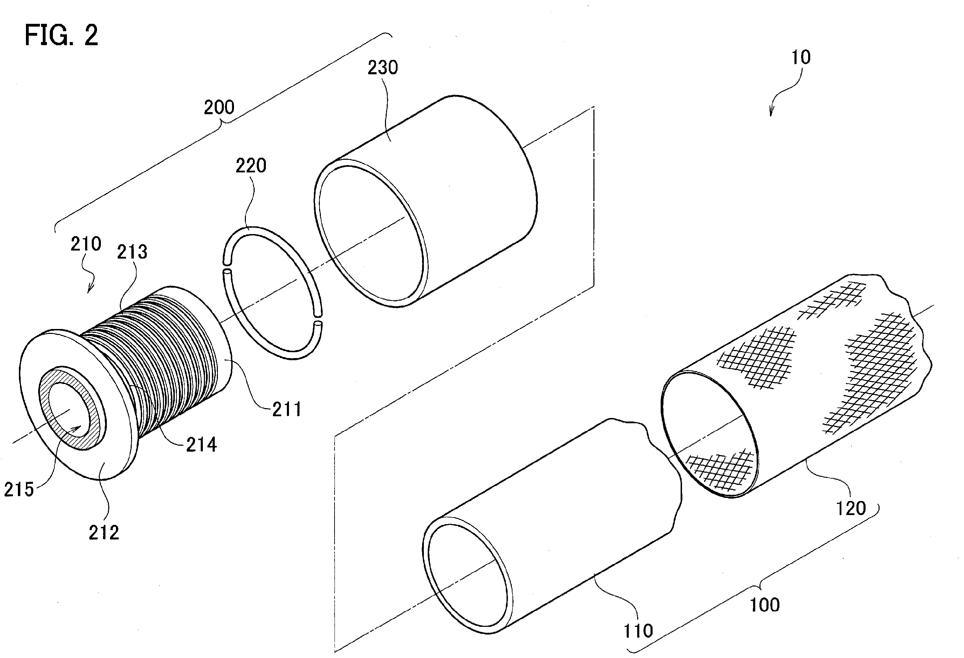

Fig. 2 is an exploded view of a part of the hydraulic actuator 10. As shown in Fig. 2, the hydraulic actuator 10 is provided with the actuator main portion 100 and the sealing mechanism 200.

As described above, the actuator main portion 100 is formed by the tube 110 and the sleeve 120.

The tube 110 is formed as a tubular body having a cylindrical shape which is expanded and contracted by pressure of fluid. The tube 110 is formed of an elastic material such as butyl rubber so as to allow the repeated contraction and expansion by fluid. Further, in a case in which the hydraulic actuator 10 is driven by oil pressure, NBR (nitrile rubber) having high oil resistance may be adopted.

The sleeve 120 is formed in a cylindrical shape to cover an outer periphery of the tube 110. The sleeve 120 is formed as a structural body in which cords oriented in a predetermined direction are braided. The cords oriented in the predetermined direction are intersected to each other so that rhombus shapes are repeatedly formed. Such a shape allows the sleeve 120 to deform like a pantograph and thereby the sleeve 120 follows the deformation of the tube 110 while restricting the contraction and the expansion of the tube 110.

As the material of the cord which forms the sleeve 120, it is preferable to adopt a fiber cord formed of aromatic polyamide (aramid fiber) or polyethylene terephthalate (PET) . However, the material of the cord is not limited to such kinds of the fiber cord, and for example, a metal cord formed of a fine filament may be adopted.

The sealing mechanism 200 is formed to seal an end portion of the actuator main portion 100 in the axial direction D_{AX}. The sealing mechanism 200 is formed by a sealing member 210, a first locking ring 220, and a caulking member 230.

The sealing member 210 includes a body portion 211 and a flange portion 212 As the material of the sealing member 210, a metal material such as stainless steel is preferably adopted, however the material of the sealing member 210 is not limited to such metal material, and a hard plastic material may be adopted.

The body portion 211 is formed in a tubular shape. A passing hole 215 through which fluid is passed is formed in the body portion 211. The passing hole 215 is communicated with a passing hole 410 (see Fig. 1). The body portion 211 is inserted into the tube 110.

The flange portion 212 is continued to the body portion 211 and is arranged at a side of an end portion in the axial direction D_{AX} of the hydraulic actuator 10 with respect to the body portion 211. An outer diameter of the flange portion 212 along the radial direction D_{R} is larger than an outer diameter of the body portion 211 along the radial direction D_{R}. The flange portion 212 is formed to lock the body portion 211 inserted into the tube 110 and the first locking ring 220.

A recess and projection portion 213 is formed on the outer periphery of the body portion 211. The recess and projection portion 213 prevents the body portion 211 inserted into the tube 110 from slipping. It is preferable that three or more projection portions are formed in the recess and projection portion 213.

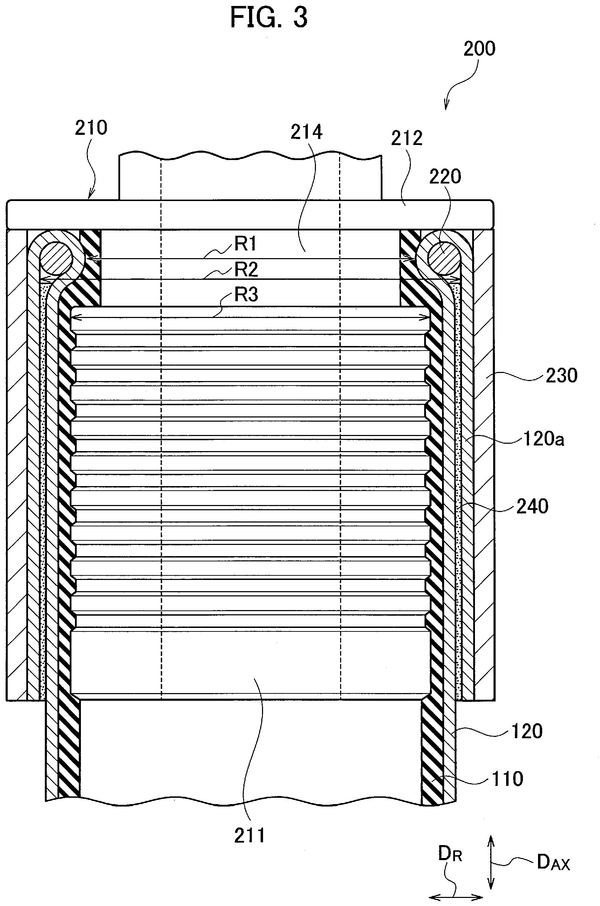

Further, a first small diameter portion 214 having an outer diameter smaller than the outer diameter of the body portion 211 is formed at a position closer to the flange portion 212 of the body portion 211. Further, a shape of the first small diameter portion 214 is further described with reference to Fig. 3 and following figures.

The first locking ring 220 is formed to lock the sleeve 120. Specifically, the sleeve 120 is folded toward an outside in the radial direction D_{R} via the first locking ring 220 (not shown in Fig. 2, and see FIG. 3).

An outer diameter of the first locking ring 220 is larger than the outer diameter of the body portion 211. The first locking ring 220 is formed to lock the sleeve 120 at a position of the first small diameter portion 214 of the body portion 211. That is, the first locking ring 220 locks the sleeve 120 at an outer side of the body portion 211 in the radial direction D_{R} and at a position adjacent to the flange portion 212.

In the present embodiment, since the first locking ring 220 is locked by the first small diameter portion 214 smaller than the body portion 211, the first locking ring 220 is formed by two divided parts. Further, the first locking ring 220 is not limited to be formed by the two divided parts, and therefore the first locking ring 220 may be formed by three or more divided parts, and a part of the divided parts may be connected in a rotatable manner.

As the material of the first locking ring 220, the metal material or the hard plastic material similar to that of the sealing member 210 can be adopted.

The caulking member 230 is formed to caulk the actuator main portion 100 in conjunction with the sealing member 210. As the material of the caulking member 230, a metal material such as aluminum alloy, brass and steel can be adopted. When the caulking member 230 is caulked by a jig for caulking the pressed mark 231 as shown in Fig. 1 is formed on the crimping member 230.

### (2) Configuration of sealing mechanism

Next, examples of the sealing mechanism 200 will be described with reference to Fig. 3 to Fig. 10.

### (2. 1) Example 1-1

Fig. 3 is a cross-sectional view of a part of the hydraulic actuator 10 along the axial direction D_{AX} of the hydraulic actuator 10 including the sealing mechanism 200 according to an example 1-1.

As described above, the sealing member 210 includes the first small diameter portion 214 having the outer diameter smaller than the outer diameter of the body portion 211.

The first locking ring 220 is arranged at an outer side of the first small diameter portion 214 in the radial direction D_{R}. An inner diameter R1 of the first locking ring 220 is smaller than an outer diameter R3 of the body portion 211. Further, an outer diameter R2 of the first locking ring 220 may be also set to be smaller than the outer diameter R3 of the body portion 211.

The body portion 211 is inserted into the tube 110 so as to be contacted with the flange portion 212. The sleeve 120 is folded toward the outer side in the radial direction D_{R} via the first locking ring 220. With this, the sleeve 120 includes a first folded portion 120a folded via the first locking ring 120.

The first folded portion 120a is adhered to the sleeve 120 located at the outer side of the tube 110 in the radial direction D_{R}, namely adhered to an unfolded part of the sleeve 120 folded by the first locking ring 220.

Specifically, an adhesive layer 240 is formed between the sleeve 120 and the first folded portion 120a. Further, the adhesive layer 240 may be formed by using an adhesive agent suitable to a kind of the cord which forms the sleeve 120.

The caulking member 230 is larger than the outer diameter of the body portion 211 of the sealing member 210. The caulking member 230 is caulked by a jig after inserted into the body portion 211. The caulking member 230 is formed to caulk the actuator main portion 100 in conjunction with the sealing member 210.

Specifically, the caulking member 230 is formed to caulk the tube 110 into which the body portion 211 is inserted, the sleeve 120 located at the outer side of the tube 110 in the radial direction D_{R}, and the first folded portion 120a. That is, the caulking member 230 caulks the tube 110, the sleeve 120, and the first folded portion 120a in conjunction with the sealing member 210.

### (2. 2) Example 1-2

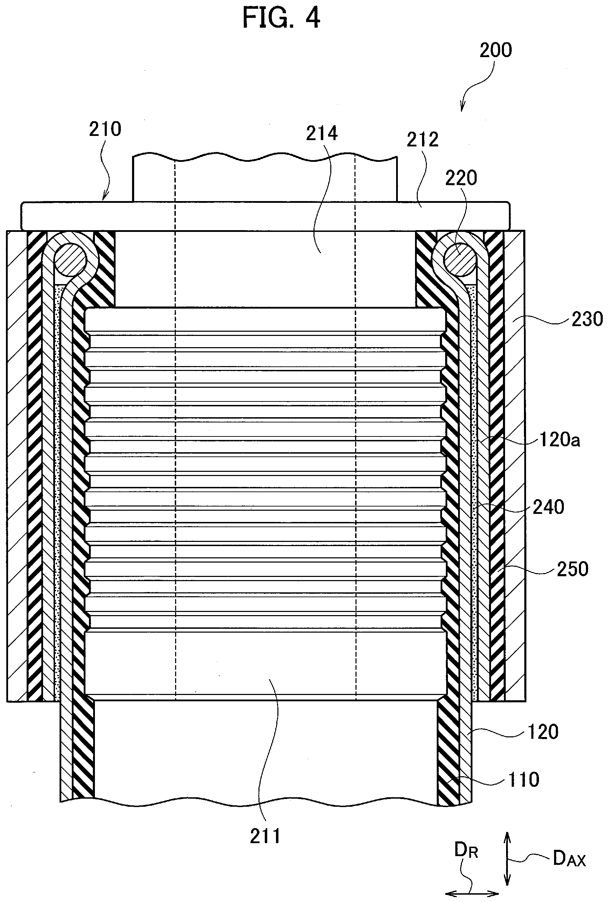

Fig. 4 is a cross-sectional view of a part of the hydraulic actuator 10 along the axial direction D_{AX} of the hydraulic actuator 10 including the sealing mechanism 200 according to an example 1-2. Hereinafter, a configuration different from the configuration of the example 1-1 is mainly described.

In the present example, an elastic member having a sheet like shape is arranged between the first folded portion 120a of the sleeve 120 and the caulking member 230.

Specifically, a rubber sheet 250 is arranged between the first folded portion 120a and the caulking member 230. The rubber sheet 250 is arranged to cover an outer periphery of the first folded portion 120a having a cylindrical shape. A kind of the rubber sheet 250 is not especially limited, however butyl rubber can be adopted similar to the tube 110.

The caulking member 230 is formed to caulk the actuator main portion 100 including the rubber sheet 250 in conjunction with the sealing member 210.

### (2. 3) Example 1-3

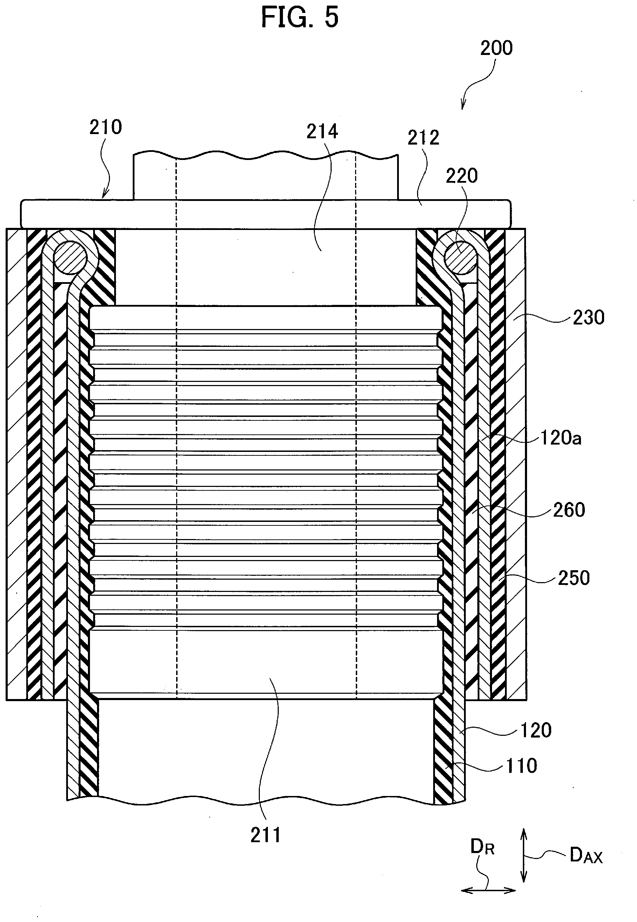

Fig. 5 is a cross-sectional view of a part of the hydraulic actuator 10 along the axial direction D_{AX} of the hydraulic actuator 10 including the sealing mechanism 200 according to an example 1-3.

In the present example, a rubber sheet 260 is adopted instead of the adhesive layer 240 in the example 1-1. The rubber sheet 260 is formed as an elastic member having a sheet like shape and is arranged between the sleeve 120 and the first folded portion 120a. The rubber which is a similar kind to the rubber sheet 250 can be used for the rubber sheet 260.

### (2. 4) Example 2-1

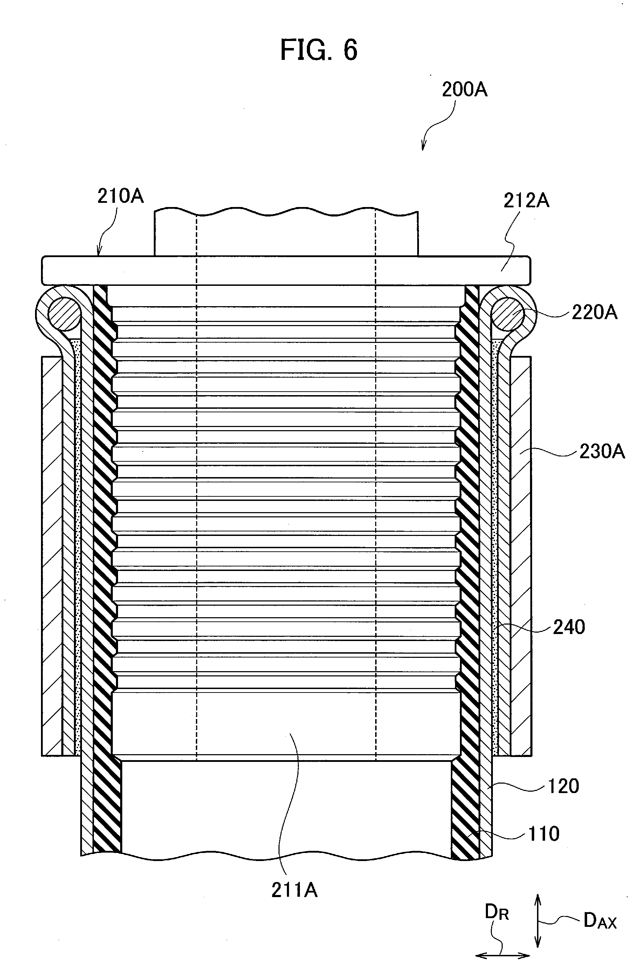

Fig. 6 is a cross-sectional view of a part of the hydraulic actuator 10 along the axial direction D_{AX} of the hydraulic actuator 10 including a sealing mechanism 200A according to an example 2-1.

In the examples 2, the sealing mechanism 200A is adopted instead of the sealing mechanism 200 in the examples 1.

The difference between the sealing mechanism 200 and the sealing mechanism 200A is whether the first small diameter portion 214 formed in the sealing member 210 is formed.

The sealing mechanism 200A is formed by a sealing member 210A, a first locking ring 220A, and a caulking member 230A.

A body portion 211A of the sealing member 210A is inserted into the tube 110. Since the first small portion 214 is not formed in the sealing member 210A contrary to the sealing member 210, an outer diameter of the first locking ring 210A is larger than an outer diameter of the body portion 211A. Thus, the first locking ring 220A is locked by the flange portion 212A and the caulking member 230A.

Further, since the outer diameter of the first locking ring 220A is larger than the outer diameter of the body portion 211A, the caulking member 230A is not contacted with the flange portion 212A. That is, a part of the folded sleeve 120 corresponding to the first locking ring 220A is exposed to the outside. Further, since the outer diameter of the first locking ring 220A is larger than the outer diameter of the body portion 211A, the first locking ring 220A may not be formed by the divided parts contrary to the first locking ring 220 in the examples 1.

Further, similar to the example 1-1, the adhesive layer 240 is formed between the sleeve 120 and the first folded portion 120a.

### (2. 5) Example 2-2

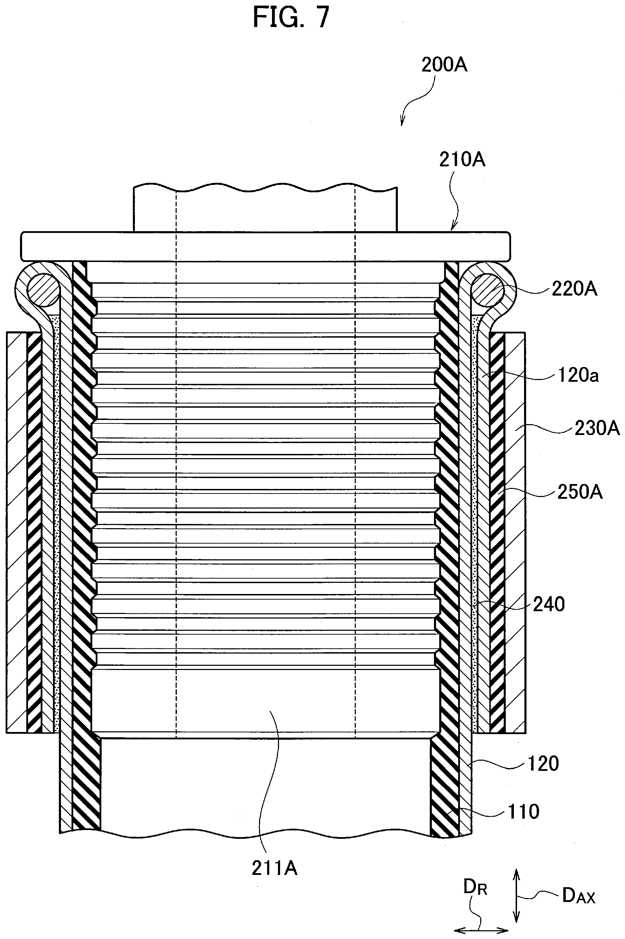

Fig. 7 is a cross-sectional view of a part of the hydraulic actuator 10 along the axial direction D_{AX} of the hydraulic actuator 10 including the sealing mechanism 200A according to an example 2-2. Hereinafter, a configuration different from the configuration of the example 2-1 is mainly described.

In the present example, an elastic member having a sheet like shape is arranged between the first folded portion 120a of the sleeve 120 and the caulking member 230A.

Specifically, a rubber sheet 250A is arranged between the first folded portion 120a and the caulking member 230A. Similar to the rubber sheet 250 in the example 1-2, the rubber sheet 250A is arranged to cover the outer periphery of the first folded portion 120a having a cylindrical shape.

### (2. 6) Example 2-3

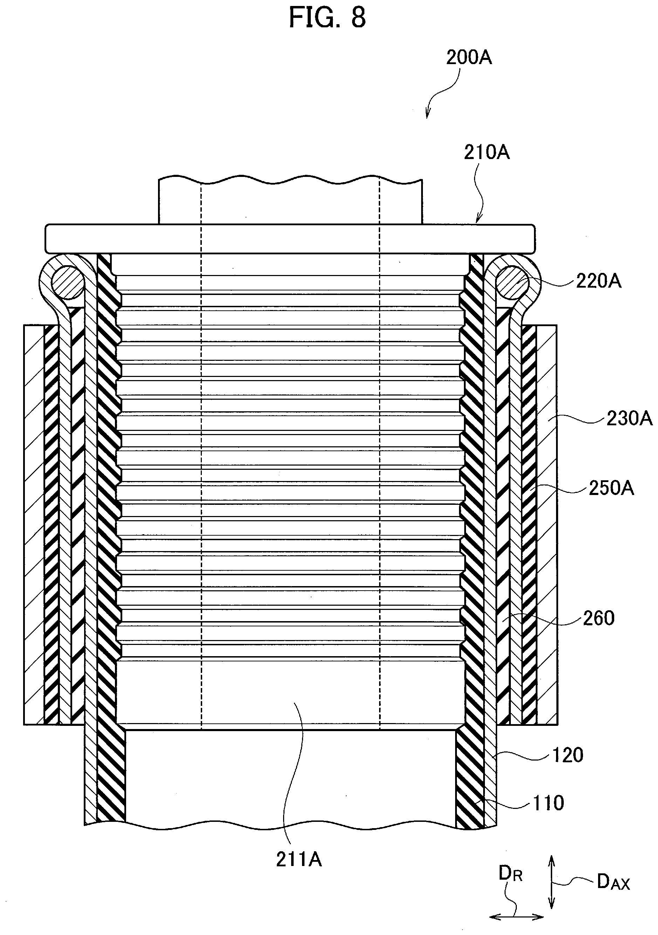

Fig. 8 is a cross-sectional view of a part of the hydraulic actuator 10 along the axial direction D_{AX} of the hydraulic actuator 10 including the sealing mechanism 200A according to an example 2-3.

In the present example, a rubber sheet 260 is adopted instead of the adhesive layer 240 in the example 2-1. Similar to the example 1-3, the rubber sheet 260 is formed as an elastic member having a sheet like shape and is arranged between the sleeve 120 and the first folded portion 120a.

### (2. 7) Example 3-1

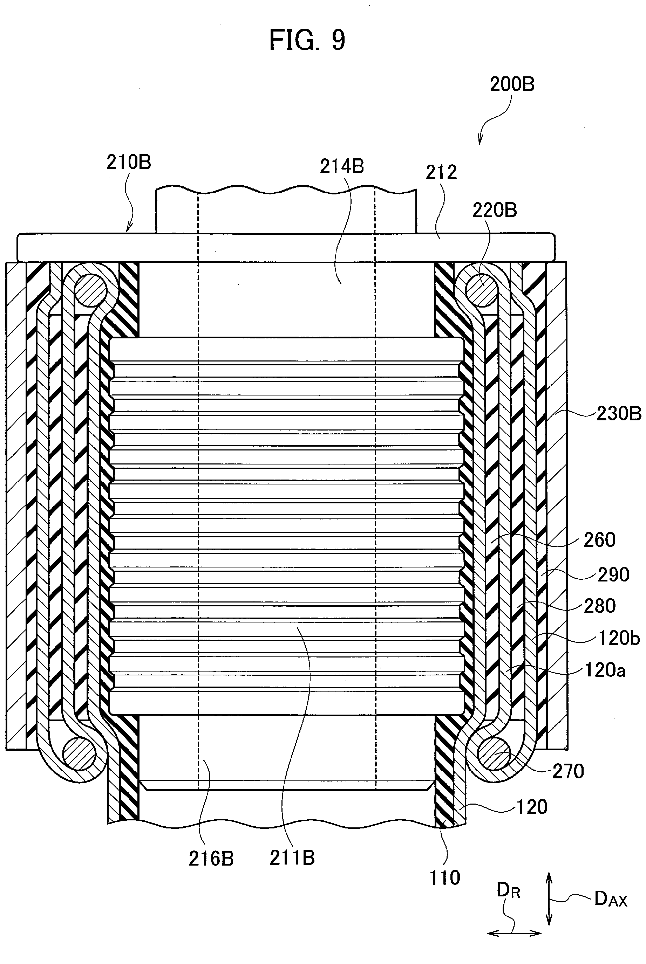

Fig. 9 is a cross-sectional view of a part of the hydraulic actuator 10 along the axial direction D_{AX} of the hydraulic actuator 10 including a sealing mechanism 200B according to an example 3-1. In the examples 3 (3-1 and 3-2), two locking rings are adopted.

As shown in Fig. 9, the sealing mechanism 200B is formed by a sealing member 210B, a first locking ring 220B, a caulking member 230B, and a second locking ring 270.

In this way, the sealing mechanism 200B includes the second locking ring 270 in addition to the first locking ring 220B. The second locking ring 270 locks the sleeve 120 at an outer side of the body portion 211B in the radial direction D_{R} and at a position closer to a center of the actuator main portion 100 in the axial direction D_{AX} than the first locking ring 220B.

Specifically, the sealing member 210B includes a second small diameter portion 216 having an outer diameter smaller than an outer diameter of the body portion 211B.

The second locking ring 270 is arranged at an outer side of the second small diameter portion 216 in the radial direction D_{R}. It is preferable that an inner diameter of the second locking ring 270 is smaller than the outer diameter of the body portion 211B. Further, an outer diameter of the second locking ring 270 may be also set to be smaller than the outer diameter of the body portion 211B. With this, the second locking ring 270 is locked by the second small diameter portion 216.

The sleeve 120 includes a second folded portion 120b folded via the second locking ring 270. The second folded portion 120b is continued to the first folded portion 120a.

That is, the sleeve 120 forms the first folded portion 120a when the sleeve 120 is folded toward the center in the axial direction D_{AX} of the actuator main portion 100 via the first locking ring 220B. Further, the sleeve 120 forms the second folded portion 120b when the first folded portion 120a is folded toward the end portion in the axial direction D_{AX} of the actuator main portion 100.

The caulking member 230B is formed to caulk the tube 110 into which the body portion 211B is inserted, the sleeve 120 located at the outer side of the tube 110 in the radial direction D_{R}, the first folded portion 120a, and the second folded portion 120b in conjunction with the sealing member 210B.

A rubber sheet 260 similar to that in the example 1-3 is arranged the sleeve 120 and the first folded portion 120a.

Further, an elastic member having a sheet like shape is arranged also between the first folded portion 120a and the second folded portion 120b. Specifically, a rubber sheet 280 is arranged between the first folded portion 120a and the second folded portion 120b. The rubber sheet 280 is arranged to cover the outer periphery of the first folded portion 120a having a cylindrical shape.

Further, a rubber sheet 290 having substantially the same shape as that of the rubber sheet 250 in the example 1-3 is arranged between the second folded portion 120b and the caulking member 230B. The rubber sheet 290 is arranged to cover an outer periphery of the second folded portion 120b having a cylindrical shape.

### (2. 8) Example 3-2

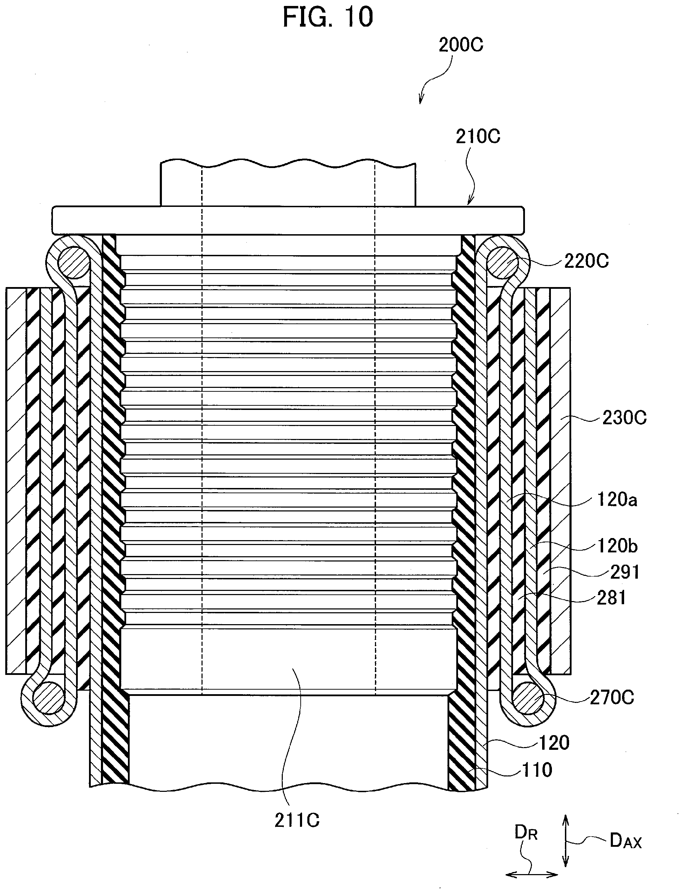

Fig. 10 is a cross-sectional view of a part of the hydraulic actuator 10 along the axial direction D_{AX} of the hydraulic actuator 10 including a sealing mechanism 200C according to an example 3-2. Hereinafter, a configuration different from the configuration of the example 3-1 is mainly described.

In the example 3-2, a sealing member 210C in which the first small diameter portion 214 and the second small diameter portion 216 are not formed is adopted.

The sealing member 210C includes a body portion 211C. Since the first small diameter portion 214 and the second small diameter portion 216 are not formed in the sealing member 210C contrary to the sealing member 210B, an outer diameter of the first locking ring 220C and an outer diameter of the second locking ring 270C are larger than an outer diameter of the body portion 211C.

The caulking member 230C is located between the first locking ring 220C and the second locking ring 270C in the axial direction D_{AX}. That is, a part of the folded sleeve 120 corresponding to the first locking ring 220C and a part of the folded sleeve 120 corresponding to the second locking ring 270C are exposed to the outside.

Further, a rubber sheet 281 having substantially the same shape as that of the rubber sheet 280 in the example 3-1 is arranged between the first folded portion 120a and the second folded portion 120b. Further, a rubber sheet 291 having substantially the same shape as that of the rubber sheet 290 in the example 3-1 is arranged between the second folded portion 120b of the sleeve 120 and the caulking member 230C.

### (3) Functions and effects

According to the embodiment described above, the following functions and effects can be obtained. The sealing mechanism 200 of the hydraulic actuator 10 is provided with the sealing member 210, the first locking ring 220, and the caulking member 230.

The first locking ring 220 locks the sleeve 120. The sleeve 120 includes the first folded portion 120a folded via the first locking ring 220. Further, the crimping member 230 caulks the tube 110, the sleeve 120 including the first folded portion 120a in conjunction with the sealing member 210.

With this, in a case in which the actuator main portion 100 is contracted or expanded by high pressure such as oil pressure, the actuator main portion 100, especially the sleeve 120, is hardly pulled off from the sealing mechanism 200.

Further, according to the configuration described above, in a case in which the oil pressure driving is adopted, leakage of oil can be firmly prevented. Further, the cord which forms the sleeve 120 is prevented from being pulled off or being cut.

That is, the hydraulic actuator 10 has sufficient durability in a case in which high pressure is applied such as a case in which the oil pressure driving is adopted.

In the present embodiment, as described in example 1-1, the sealing member 210 includes the first small diameter portion 214, and the inner diameter R1 of the first locking ring 220 is smaller than the outer diameter R3 of the body portion 211. With this, since the first locking ring 220 is locked by the body portion 211, the actuator main portion 100 is further hardly pulled off.

Further, in the present embodiment, as described in the examples 3-1 and 3-2, the sleeve 120 includes the second folded portion 120b folded by the second locking ring 270 (or the second locking ring 270C). With this, since the first folded portion 120a and the second folded portion 120b are caulked by the caulking member 230B (or the caulking member 230C), the actuator main portion 100 is further hardly pulled off.

Further, when the actuator main portion 100, specifically the tube 110, is expanded, the tube 110 is deformed along the second locking ring 270, and thereby the tube 110 can be prevented from deforming in a sharp angle manner. With this, the tube 110 can be prevented from being broken.

In the present embodiment, as described in the example 3-1, the inner diameter of the second locking ring 270 is smaller than the outer diameter of the body portion 211B. With this, since the second locking ring 270 is locked by the body portion 211B, the actuator main portion 100 is further hardly pulled off.

In the present embodiment, as described in the examples 1-2, 2-1 and 2-2, the first folded portion 120a is adhered to the sleeve 120 located at the outer side of the tube 110 in the radial direction D_{R} by the adhesive layer 240. With this, since the sleeve 120 and the first folded portion 120a are hardly peeled from each other, the actuator main portion 100 is further hardly pulled off.

In the present embodiment, as described in the examples 1-3 or the like, the rubber sheet 260 is arranged between the sleeve 120 located at the outer side of the tube 110 in the radial direction D_{R}, and the first folded portion 120a. Further, the rubber sheet 250 is arranged between the first folded portion 120a and the caulking member 230. Further, as described in the examples 3-1 and 3-2, the rubber sheet 280 (or the rubber sheet 281) is arranged between first folded portion 120a and the second folded portion 120b.

With this, since the rubber sheet works as a cushion layer, the cord which forms the first folded portion 120a and the second folded portion 120b can be further firmly prevented from being cut.

### (4) Modification

The present invention is described above with reference to the examples, however the present invention is not limited to such descriptions, and it is obvious for a skilled person that the present invention can be modified or improved in various ways.

For example, in the embodiment described above, the sleeve 120 is formed of the organic fiber cord or the metal cord. A surface of such a cord may be coated by rubber. Further, the sleeve 120 is not limited to the structure in which the cords oriented in the predetermined direction are braided, as long as the sleeve 120 can control the contraction and the expansion of the tube 110 within a predetermined range.

Further, in the examples described above, the adhesive layer 240 or the rubber sheet 260 is arranged between the sleeve 120 and the first folded portion 120a, however it may be determined to adopt either of them in accordance with durability required in the hydraulic actuator 10 and the material of the sleeve 120. That is, the adhesive layer 240 is not necessarily arranged, and therefore the first folded portion 120a may not be adhered to an unfolded part of the sleeve 120.

### Second embodiment

Next, a second embodiment of the hydraulic actuator will be described. Hereinafter, a configuration different from the configuration of the first embodiment described above is mainly described, and therefore a description of a similar part is omitted as needed.

Also in the present embodiment, a configuration of the hydraulic actuator is similar to the configuration of the hydraulic actuator 10 shown in Fig. 1 to Fig. 3. However, a configuration of a actuator main portion 100 is different.

### (1) Configuration of actuator main portion 100

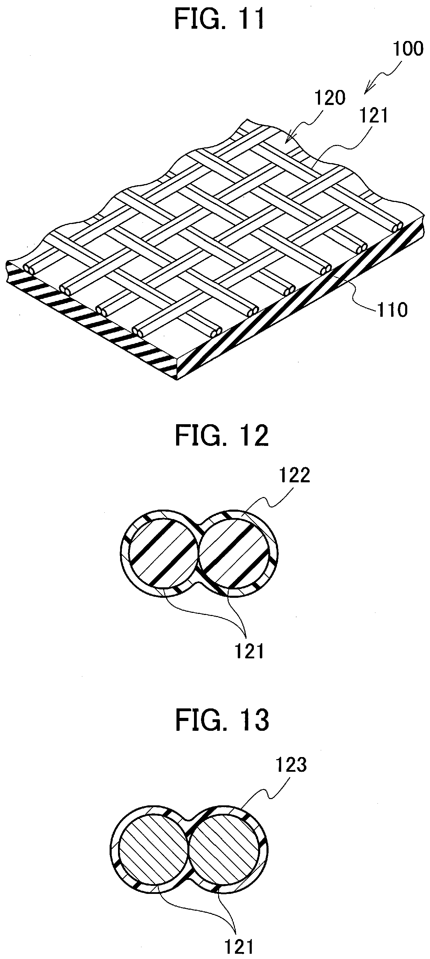

Fig. 11 is a developed view of a part of the actuator main portion 100. As shown in Fig. 11, the actuator main portion 100 is formed by a tube 110 and a sleeve 120.

The tube 110 is formed as a tubular body (a pipe body) formed of an elastic material such as butyl rubber. Further, in a case in which the hydraulic actuator 10 is driven by oil pressure, NBR (nitrile rubber) having high oil resistance may be adopted.

The sleeve 120 is arranged to cover an outer periphery of the tube 110. The sleeve 120 is formed by braiding cords 121 oriented to be intersected with each other.

Fig. 12 is a cross-sectional view along a radial direction of the cord 121. As shown in Fig. 12, the sleeve 120 includes a coating layer 122 which coats a surface of the cord 121.

Here, in Fig. 11 and Fig. 12, two cords 121 are arranged as a pair, and pairs of the cords 121 are oriented to be intersected with each other, however the number of the cords 121 forming a set may be other than two (monofilament or multifilament). In a case in which the number of the cords 121 is more than two, the cords 121 may be twisted.

The cord 121 is formed by an organic fiber cord. Specifically, it is preferable that a fiber cord formed of aromatic polyamide (aramid fiber) or polyethylene terephthalate (PET) is adopted as the cord 121. However, an organic fiber cord other than such a fiber cord may be adopted. Further, a diameter of the fiber cord is not especially limited as long as the fiber cord does not interrupt the operation of the actuator main portion 100.

Further, pretreatment using a reactive agent such as epoxy resin may be applied to the cord 121 in order to enhance adhesiveness of the cord 121 to an aqueous mixture solution which forms the coating layer 122.

The coating layer 122 is formed by a mixture of thermosetting resin and latex. Specifically, the coating layer 122 is formed by coating the cord 121 with an aqueous mixture solution of the thermosetting resin and the latex and then drying and heat curing the aqueous mixture solution.

It is preferable that the coating layer 122 is formed by using the aqueous mixture solution in which the solid fraction (wt%) of the thermosetting resin and the latex is set in a range between 15% and 50%. More preferably, the solid fraction is set in a range between 20% and 40%.

Examples of the thermosetting resin include phenol resin, resorcinol resin, and urethane resin. Or alternatively, a mixture of several resins thereof may be adopted.

Examples of the latex include VP (styrene-butadiene-vinyl pyridine copolymer) latex, SBR (low styrene-butadiene copolymer) latex, NBR (butadiene-acrylonitrile copolymer) latex, and a mixture of several latexes thereof.

### (2) Functions and effects

Hereinafter, functions and effects of the hydraulic actuator 10 according to the present embodiment described above will be described.

Table 1 shows configurations of the hydraulic actuators according to comparative examples and examples, and evaluation test result thereof.

**[Table 1]**

| | | Comparative example 1 | Comparative example 2 | Comparative example 3 | Example 1 | Example 2 | Example 3 |
|---|---|---|---|---|---|---|---|
| Configuration | Coating layer (resin) | Resorcinol resin | None | Urethane resin | Resorcinol resin | Urethane resin | Resorcinol-urethane resin |
| | Coating layer (latex) | SBR | None | NR | VP | NBR | NBR-VP |
| | Solid fraction (wt%) | 10 | - | 60 | 20 | 40 | 30 |
| Test result | Durability | 100 | 90 | 95 | 110 | 110 | 120 |

As shown in Table 1, the hydraulic actuators having different coating layers according to the comparative examples 1 to 3 and the examples 1 to 3 are manufactured, and the evaluation test relating to the durability of the hydraulic actuators is executed.

In each of the comparative example 1 and the comparative example 3, the solid fraction (wt%) of the aqueous mixture solution of the thermosetting resin and the latex used for forming the coating layer is out of the range between 15% and 50% (preferably between 20% and 40%). Further, in the comparative example 2, the latex is not used for the coating layer. On the other hand, in the examples 1 to 3, the solid fraction (wt%) of the aqueous mixture solution is in a range between 20% and 40%.

In the evaluation test relating to the durability, each of the hydraulic actuators is driven by oil pressure and then the operation number (time) until damage is generated in the actuator main portion is measured. The values in the field of "Durability" indicate index numbers as the measurement result of the comparative example 1 is set to 100.

In the examples 1 to 3, the mixture of the thermosetting resin and the latex is used for the coating layer and the solid fraction thereof is in the range between 20% and 40%, and thereby the durability is enhanced drastically.

In this way, according to the hydraulic actuator 10, the cord 121 is formed of the organic fiber and the coating layer 122 which coats the surface of the cord 121 is formed of the mixture of the thermosetting resin and the latex.

In the McKibben type hydraulic actuator such as the hydraulic actuator 10, the durability when the contraction and the expansion of the actuator main portion 100 are repeated may become a problem due to the damage caused by the friction between the organic fiber cord 121 and the tube 110 or the friction of the cord 121 itself.

According to the hydraulic actuator 10, since the surface of the cord 121 is coated by the coating layer 122 formed of the mixture of the thermosetting resin and the latex, the cord 121 can be prevented from being damaged and the friction coefficient of the surface of the cord 121 can be appropriately decreased.

That is, the hydraulic actuator 10 has sufficient durability in a case in which high pressure is applied such as a case in which oil pressure driving is adopted.

In the present embodiment, the coating layer 122 may be formed by using the aqueous mixture solution in which the solid fraction (wt%) of the thermosetting resin and the latex is set in a range between 15% and 50% (preferably between 20% and 40%) . Further, the thermosetting resin is formed of phenol resin, resorcinol resin, urethane resin, or a mixture of several resins thereof. Further, the latex is formed of VP latex, SBR latex, NBR latex, or a mixture of several latexes thereof.

With this, the surface of the cord 121 formed of the organic fiber can be coated easily and firmly, and the friction coefficient of the surface of the cord 121 can be decreased appropriately.

In the present embodiment, the sealing member 210 includes the rubber sheet 250 and the rubber sheet 260 contacted with the sleeve 120. By contacting the sleeve 120 formed of the cord 121 which is coated by the coating layer 122 in such a way, an effect of preventing the sleeve 120 (the cord 121) having the friction coefficient being decreased from being pulled off from the sealing member 210 can be obtained.

Especially, since the cord 121 is coated by the coating layer 122 formed of the mixture of the thermosetting resin and the latex, the coating layer 122 and the rubber sheet 260 are adhered to each other, and therefore the excellent effect of the preventing the sleeve 120 from being pulled off can be obtained.

### Third embodiment

Next, a third embodiment of the hydraulic actuator will be described. Hereinafter, a configuration different from the configuration of the second embodiment described above is mainly described, and therefore a description of a similar part is omitted as needed.

Also in the present embodiment, a configuration of the actuator main portion 100 is similar to the configuration of the actuator main portion 100 shown in Fig. 4. However, a configuration of the cord 121 which forms the actuator main portion 100 is different.

Fig. 13 is a cross-sectional view along a radial direction of the cord 121. As shown in Fig. 13, the sleeve 120 includes a coating layer 123 which coats a surface of the cord 121.

The cord 121 is formed of steel, namely alloy including iron as a main component. It is preferable that a diameter of the cord 121 is equal to or less than 0.2 mm. Since the strength of the cord 121 is required, the steel of 70C or more with much carbon content is preferably adopted.

The coating layer 123 is formed of thermoplastic resin, thermosetting resin, or a mixture of thermoplastic resin and thermosetting resin.

Specifically, as the coating layer 123, olefin-based thermoplastic elastomer (TPE), polyester-based thermoplastic elastomer (TPE), polypropylene (PP), polyethylene (PE), polyvinyl chloride (PVC), polyamide (PA), phenol resin, epoxy resin, or urethane resin is preferably adopted. Or alternatively, a mixture of several resins thereof may be adopted.

The thermoplastic resin which forms the coating layer 123 is preferably adhered to the cord 121. Further, resin modified by Maleic acid or the like may be adopted in order to enhance the adhesiveness to the cord 121.

Further, the caulking member 230 (see Fig. 3) is preferably formed of thermoplastic resin, thermosetting resin, or a mixture of the thermoplastic resin and the thermosetting resin, which is the same as the coating layer 123. That is, the caulking member 230 is preferably formed of the same resin material as that of the coating layer 123. Further, in a case in which the caulking member 230 is formed of the same thermoplastic resin as the coating layer 123, the adhesive layer 240 may not be formed.

Further, the adhesive layer 240 may include the same resin as the coating layer 123. Further, a sheet like member including the same resin as the coating layer 123 may be intervened between caulking member 230 and the first folded portion 120a.

### (2) Functions and effects

Hereinafter, functions and effects of the hydraulic actuator 10 according to the present embodiment described above is described.

Table 2 shows configurations of the hydraulic actuators according to comparative examples and examples, and evaluation test result thereof.

**[Table 2]**

| | | Comparative example 1 | Comparative example 2 | Example 1 | Example 2 | Example 3 |
|---|---|---|---|---|---|---|
| Configuration | Coating layer-resin | None | None | Urethane | PP | PA |
| | Kind of cord | Steel | Steel | Steel | Steel | Steel |
| | Diameter of cord (mm) | 0.18 | 0.30 | 0.18 | 0.18 | 0.18 |
| Test result | Durability | 100 | 90 | 115 | 110 | 110 |

As shown in Table 2, the hydraulic actuators according to the comparative examples 1 and 2 and the examples 1 to 3 are manufactured, and the evaluation test relating to the durability of the hydraulic actuators is executed.

Each of the hydraulic actuators according to the comparative examples 1 and 2 does not include the coating layer. On the other hand, each of the examples 1 to 3 includes the coating layer which coats the cord, and the diameter of the cord is equal to or less than 0.2 mm (0.18 mm) .

In the evaluation test relating to the durability, each of the hydraulic actuators is driven by oil pressure and the operation number (time) until damage is generated in the actuator main portion is measured. The values in the field of "Durability" indicate index numbers as the measurement result of the comparative example 1 is set to 100.

Each of the examples 1 to 3 includes the coating layer and the diameter of the cord is equal to or less than 0.2 mm, and thereby the durability is enhanced drastically.

In this way, according to the hydraulic actuator 10, the cord 121 is formed of steel, and the coating layer 123 which coats the cord 121 is formed of the thermoplastic resin, the thermosetting resin, or the mixture of the thermoplastic resin and the thermosetting resin.

Generally, in the McKibben type hydraulic actuator like the hydraulic actuator 10, an organic fiber cord is widely adopted. However, since the cord 121 is formed of steel, the strength thereof is extremely high. Thus, the hydraulic actuator 10 has sufficient strength in a case in which high pressure is applied due to the oil pressure driving.

Here, the durability when the contraction and the expansion of the actuator main portion 100 are repeated may become a problem due to the friction between the cord 121 and the tube 110. Further, decrease of the strength due to rust of the cord 121 may also become a problem.

In the hydraulic actuator 10, since the cord 121 is coated by the coating layer 123, the rust of the cord 121 can be prevented. Further, the friction coefficient of the surface of the cord 121 is decreased, and thereby the cord 121 is slipped easily. In this way, according to the hydraulic actuator 10, the durability is also enhanced.

That is, the hydraulic actuator 10 has sufficient durability in a case in which high pressure is applied such as a case in which oil pressure driving is adopted.

In the present embodiment, the coating layer 123 is formed of olefin-based thermoplastic elastomer (TPE), polyester-based thermoplastic elastomer (TPE), polypropylene (PP), polyethylene (PE), polyvinyl chloride (PVC), polyamide (PA), phenol resin, epoxy resin, urethane resin, or a mixture of several resins thereof. By adopting such thermoplastic resin, the surface of the cord 121 formed of steel can be coated easily and firmly.

In the present embodiment, the caulking member 230 can be formed of the thermoplastic resin, the thermosetting resin, or the mixture of the thermoplastic resin and the thermosetting resin, which is the same as the coating layer 123. In this way, by caulking the actuator main portion 100 by using resin for cushion having compatibility with the resin of the coating layer 123, the adhesiveness of the cord 121 can be enhanced.

That is, the caulking member 230 and the actuator main portion 100 (the sleeve 120) can be integrated with each other. With this, the durability of the hydraulic actuator 10 can be enhanced, and especially an effect of preventing the actuator main portion 100 from being pulled off from the sealing member 210 can be obtained.

In the present embodiment, the diameter of the cord 121 is equal to or less than 0.2 mm. With this, sufficient flexibility of the sleeve 120 can be secured, and smooth operation of the hydraulic actuator 10 can be achieved.

### Other embodiments

The invention according to the embodiments described above may be described as below. A hydraulic actuator (the hydraulic actuator 10) according to one aspect of the present invention includes an actuator main portion (the actuator main portion 100) formed by a tube (the tube 110) having a cylindrical shape which is expanded and contracted by pressure of fluid and a sleeve (the sleeve 120) formed as a structural body in which cords oriented in a predetermined direction are braided, the sleeve being formed to cover an outer periphery of the tube, and a sealing mechanism (for example, the sealing mechanism 200) which seals an end portion of the actuator main portion in an axial direction. The sealing mechanism includes a sealing member (for example, the sealing member 210) having a body portion (the body portion 211) and a flange portion (the flange portion 212), a caulking member (the caulking member 230) which caulks the actuator main portion in conjunction with the sealing member, and a first locking ring (the first locking ring 220) which locks the sleeve. The tube inserted into the body portion. The flange portion is continued to the body portion and an outer diameter of the flange portion along a radial direction of the actuator main portion is larger than an outer diameter of the body portion. The first locking ring locks the sleeve at an outer side of the body portion in the radial direction. The sleeve includes a first folded portion (the first folded portion 120a) folded via the first locking ring. The caulking member caulks the tube into which the body portion is inserted, the sleeve located at the outer side of the tube in the radial direction, and the first folded portion in conjunction with the sealing member.

In the one aspect of the present invention, the sealing member may include a first small diameter portion (the first small diameter portion 214) having an outer diameter smaller than the outer diameter of the body portion, and the first locking ring may be arranged at an outer side of the first small diameter portion in the radial direction, and at least an inner diameter of the first locking ring may be smaller than the outer diameter of the body portion.

In the one aspect of the present invention, the sealing mechanism may include a second locking ring (for example, the second locking ring 270) which locks the sleeve at the outer side of the body portion in the radial direction and at a position closer to a center of the actuator main portion in the axial direction than the first locking ring, and the sleeve may include a second folded portion (the second folded portion 120b) folded via the second locking ring, and the second folded portion may be continued to the first folded portion, and the caulking member may caulk the tube into which the body portion is inserted, the sleeve located at the outer side of the tube in the radial direction, the first folded portion, and the second folded portion in conjunction with the sealing member.

In the one aspect of the present invention, the sealing member may include a second small diameter portion (the second small diameter portion 216) having an outer diameter smaller than the outer diameter of the body portion, and the second locking ring may be located at an outer side of the second small diameter in the radial direction, and at least an inner diameter of the second locking ring may be smaller than the outer diameter of the body portion.

In the one aspect of the present invention, the first folded portion may be adhered to the sleeve located at an outer side of the tube in the radial direction.

In the one aspect of the present invention, an elastic member (the rubber sheet 260) having a sheet like shape may be arranged between the sleeve located at an outer side of the tube in the radial direction and the first folded portion.

In the one aspect of the present invention, an elastic member (for example, the rubber sheet 250) having a sheet like shape may be arranged between the first folded portion and the caulking member.

In the one aspect of the present invention, an elastic member (for example, the rubber sheet 280) having a sheet like shape may be arranged between the first folded portion and the second folded portion.

In the one aspect of the present invention, the cord may be formed of organic fiber, the hydraulic actuator may include a coating layer which coats an surface of the cord, and the coating layer may be formed of a mixture of thermosetting resin and latex.

In the one aspect of the present invention, the coating layer may be formed by using an aqueous mixture solution in which a solid fraction (wt%) of the thermosetting resin and the latex is in a range between 15% and 50%.

In the one aspect of the present invention, the thermosetting resin may be formed of phenol resin, resorcinol resin, urethane resin, or a mixture of several resins thereof.

In the one aspect of the present invention, the latex may be formed of VP latex, SBR latex, NBR latex, or a mixture of several latexes thereof.

In the one aspect of the present invention, the sealing mechanism may include a rubber sheet (for example, the rubber sheet 250) contacted with the sleeve.

In the one aspect of the present invention, the cord may be formed of steel, and the hydraulic actuator may include a coating layer (the coating layer 123) which coats an surface of the cord, and the coating layer may be formed of thermoplastic resin, thermosetting resin, or a mixture of the thermoplastic resin and the thermosetting resin.

In the one aspect of the present invention, the coating layer may be formed of olefin-based thermoplastic elastomer (TPE), polyester-based thermoplastic elastomer (TPE), polypropylene (PP), polyethylene (PE), polyvinyl chloride (PVC), polyamide (PA), phenol resin, epoxy resin, or urethane resin, or a mixture of several resins thereof.

As described above, the embodiments of the present invention are described, however the present invention is not limited to the description and the drawings forming a part of the present disclosure. Various modifications, examples, and operation techniques will be apparent from the present disclosure to a person skilled in the art.

### [Industrial Applicability]

The hydraulic actuator according to one aspect of the present invention has sufficient durability in a case in which high pressure is applied such as a case in which oil pressure driving is adopted.

### [Reference Signs List]

- 10:: hydraulic actuator
- 20:: connection portion
- 100:: actuator main portion
- 110:: tube
- 120:: sleeve
- 120a:: first folded portion
- 120b:: second folded portion
- 121:: cord
- 122:: coating layer
- 123:: coating layer
- 200, 200A, 200B, 200C:: sealing mechanism
- 210, 210A, 210B, 210C:: sealing member
- 211, 211A, 211B, 211C:: body portion
- 212, 212A:: flange portion
- 213:: recess and projection portion
- 214:: first small diameter portion
- 215:: passing hole
- 216:: second small diameter portion
- 220, 220A, 220B, 220C:: first locking ring
- 230, 230A, 230B, 230C:: caulking member
- 231:: pressed mark
- 240:: adhesive layer
- 250, 250A:: rubber sheet
- 260:: rubber sheet
- 270, 270C:: second locking ring
- 280, 281:: rubber sheet
- 290, 291:: rubber sheet
- 300:: sealing mechanism
- 400:: fitting
- 410:: passing hole

## Claims

1. A hydraulic actuator (10) comprising:
an actuator main portion (100) formed by a tube (110) having a cylindrical shape which is expanded and contracted by pressure of fluid, and a sleeve (120) formed as a structural body in which cords (121) oriented in a predetermined direction are braided, the sleeve (120) being configured to cover an outer periphery of the tube (110); and
a sealing mechanism (200, 200A, 200B, 200C) which seals an end portion of the actuator main portion (100) in an axial direction,
wherein:
the sealing mechanism (200, 200A, 200B, 200C) comprises:
a sealing member (210, 210A, 210B, 210C) having a body portion (211, 211A, 211B, 211C) and a flange portion (212, 212A);
a caulking member (230, 230A, 230B, 230C) which caulks the actuator main portion (100) in conjunction with the sealing member (210, 210A, 210B, 210C); and
a first locking element (220) which locks the sleeve (120);
the body portion (211, 211A, 211B, 211C) inserted into the tube (110);
the flange portion (212, 212A) is continued to the body portion (211, 211A, 211B, 211C) and an outer diameter of the flange portion (212, 212A) along a radial direction of the actuator main portion (100) is larger than an outer diameter (R3) of the body portion (211, 211A, 211B, 211C);
the first locking element (220) locks the sleeve (120) at an outer side of the body portion (211, 211A, 211B, 211C) in the radial direction;
**characterised in that**
the first locking element (220) is a first locking ring (220) and
the sleeve (120) comprises a first folded portion (120a) folded via the first locking ring (220); and
the caulking member (230, 230A, 230B, 230C) caulks the tube (110) into which the body portion (211, 211A, 211B, 211C) is inserted, the sleeve (120) located at the outer side of the tube (110) in the radial direction, and the first folded portion (120a) in conjunction with the sealing member (210, 210A, 210B, 210C).

2. The hydraulic actuator (10) according to claim 1, wherein:
the sealing member (210, 210B) comprises a first small diameter portion (214) having an outer diameter smaller than the outer diameter of the body portion (211, 211B);
the first locking ring (220) is arranged at an outer side of the first small diameter portion (214) in the radial direction; and
at least an inner diameter (R1) of the first locking ring (220) is smaller than the outer diameter (R3) of the body portion (211, 211B).

3. The hydraulic actuator (10) according to claim 1, wherein:
the sealing mechanism (200B, 200C) comprises a second locking ring (270, 270C) which locks the sleeve (120) at the outer side of the body portion (211B, 211C) in the radial direction and at a position closer to a center of the actuator main portion (100) in the axial direction than the first locking ring (220);
the sleeve (120) comprises a second folded portion (120b) folded via the second locking ring (270, 270C);
the second folded portion (120b) is continued to the first folded portion (120a); and
the caulking member (230, 230A, 230B, 230C) caulks the body portion (211B, 211C) inserted into the tube (110), the sleeve (120) located at the outer side of the tube (110) in the radial direction, the first folded portion (120a), and the second folded portion (120b) in conjunction with the sealing member (210B, 210C).

4. The hydraulic actuator (10) according to claim 3, wherein:
the sealing member (210B) comprises a second small diameter portion (216) having an outer diameter smaller than the outer diameter of the body portion (211B);
the second locking ring (270) is located at an outer side of the second small diameter portion (216) in the radial direction; and
at least an inner diameter of the second locking ring (270) is smaller than the outer diameter of the body portion (211B).

5. The hydraulic actuator (10) according to claim 1, wherein the first folded portion (120a) is adhered to the sleeve (120) located at an outer side of the tube (110) in the radial direction.

6. The hydraulic actuator (10) according to claim 1, wherein an elastic member having a sheet like shape is arranged between the sleeve (120) located at an outer side of the tube (110) in the radial direction and the first folded portion (120a).

7. The hydraulic actuator (10) according to claim 1, wherein an elastic member (250, 250A) having a sheet like shape is arranged between the first folded portion (120a) and the crimping member (230, 230A, 230B, 230C).

8. The hydraulic actuator (10) according to claim 3, wherein an elastic member having a sheet like shape is arranged between the first folded portion (120a) and the second folded portion (120b).

9. The hydraulic actuator (10) according to claim 1, wherein:
the cord (121) is formed of organic fiber;
the hydraulic actuator (10) comprises a coating layer (122, 123) which coats a surface of the cord (121); and
the coating layer (122, 123) is formed of a mixture of thermosetting resin and latex.

10. The hydraulic actuator (10) according to claim 9, wherein the coating layer (122, 123) is formed by using an aqueous mixture solution in which a solid fraction (wt%) of the thermosetting resin and the latex is in a range between 15% and 50%.

11. The hydraulic actuator (10) according to claim 9 or 10, wherein the thermosetting resin is formed of phenol resin, resorcinol resin, urethane resin, or a mixture of several resins thereof.

12. The hydraulic actuator (10) according to any one of claims 9 to 11, wherein the latex is formed of VP latex, SBR latex, NBR latex, or a mixture of several latexes thereof.

13. The hydraulic actuator (10) according to any one of claims 9 to 12, wherein the sealing mechanism comprises a rubber sheet contacted with the sleeve (120).

14. The hydraulic actuator (10) according to claim 1, wherein:
the cord (121) is formed of steel;
the hydraulic actuator (10) comprises a coating layer (122, 123) which coats a surface of the cord (121); and
the coating layer (122, 123) is formed of thermoplastic resin, thermosetting resin, or a mixture of the thermoplastic resin and the thermosetting resin.

15. The hydraulic actuator (10) according to claim 14, wherein the coating layer (122, 123) is formed of olefin-based thermoplastic elastomer (TPE), polyester-based thermoplastic elastomer (TPE), polypropylene (PP), polyethylene (PE), polyvinyl chloride (PVC), polyamide (PA), phenol resin, epoxy resin, or urethane resin, or a mixture of several resins thereof.

## Patentansprüche

1. Hydraulikaktor (10), der Folgendes umfasst:
einen Aktor-Hauptabschnitt (100), der durch eine Röhre (110) gebildet wird, die eine zylindrische Form aufweist, die durch Druck eines Fluids ausgedehnt und zusammengezogen wird, und eine Hülse (120), die als ein Strukturkörper geformt ist, in dem Kords (121), die in einer vorbestimmten Richtung ausgerichtet sind, verflochten sind, wobei die Hülse (120) dafür konfiguriert ist, einen äußeren Umfang der Röhre (110) abzudecken, und
einen Abdichtungsmechanismus (200, 200A, 200B, 200C), der einen Endabschnitt des Aktor-Hauptabschnitts (100) in einer axialen Richtung abdichtet,
wobei:
der Abdichtungsmechanismus (200, 200A, 200B, 200C) Folgendes umfasst:
ein Abdichtungselement (210, 210A, 210B, 210C), das einen Körperabschnitt (211, 211A, 211B, 211C) und einen Flanschabschnitt (212, 212A) aufweist,
ein Verstemmungselement (230, 230A, 230B, 230C), das den Aktor-Hauptabschnitt (100) in Verbindung mit dem Abdichtungselement (210, 210A, 210B, 210C) verstemmt, und
ein erstes Arretierungselement (220), das die Hülse (120) arretiert,
der Körperabschnitt (211, 211A, 211B, 211C) in die Röhre (110) eingesetzt ist,
der Flanschabschnitt (212, 212A) zu dem Körperabschnitt (211, 211A, 211B, 211C) fortgesetzt ist und ein Außendurchmesser des Flanschabschnitts (212, 212A) entlang einer radialen Richtung des Aktor-Hauptabschnitts (100) größer ist als ein Außendurchmesser (R3) des Körperabschnitts (211, 211A, 211B, 211C),
das erste Arretierungselement (220) die Hülse (120) an einer in der radialen Richtung äußeren Seite des Körperabschnitts (211, 211A, 211B, 211C) arretiert,
**dadurch gekennzeichnet, dass**
das erste Arretierungselement (220) ein erster Arretierungsring (220) ist und die Hülse (120) einen ersten gefalteten Abschnitt (120a) umfasst, der über den ersten Arretierungsring (220) gefaltet ist, und
das Verstemmungselement (230, 230A, 230B, 230C) die Röhre (110) verstemmt, in die der Körperabschnitt (211, 211A, 211B, 211C) eingesetzt ist, wobei die Hülse (120) an der in der radialen Richtung äußeren Seite der Röhre (110) angeordnet ist und der erste gefaltete Abschnitt (120a) in Verbindung mit dem Abdichtungselement (210, 210A, 210B, 210C) ist.

2. Hydraulikaktor (10) nach Anspruch 1, wobei:
das Abdichtungselement (210, 210B) einen ersten Abschnitt (214) mit kleinem Durchmesser umfasst, der einen Außendurchmesser aufweist, der kleiner ist als der Außendurchmesser des Körperabschnitts (211, 211B),
der erste Arretierungsring (220) an einer in der radialen Richtung äußeren Seite des ersten Abschnitts (214) mit kleinem Durchmesser angeordnet ist und
wenigstens ein Innendurchmesser (R1) des ersten Arretierungsrings (220) kleiner ist als der Außendurchmesser (R3) des Körperabschnitt (211, 211B).

3. Hydraulikaktor (10) nach Anspruch 1, wobei:
der Abdichtungsmechanismus (200B, 200C) einen zweiten Arretierungsring (270, 270C) umfasst, der die Hülse an der in der radialen Richtung äußeren Seite des Körperabschnitts (211B, 211C) und an einer Position, näher zu einer Mitte des Aktor-Hauptabschnitts (100) in der axialen Richtung als der erste Arretierungsring (220), arretiert,
die Hülse (120) einen zweiten gefalteten Abschnitt (120b) umfasst, der über den zweiten Arretierungsring (270, 270C) gefaltet ist,
der zweite gefaltete Abschnitt (120b) bis zum ersten gefalteten Abschnitt (120a) verläuft, und
das Verstemmungselement (230, 230A, 230B, 230C) den Körperabschnitt (211B, 211C) verstemmt, der in die Röhre (110) eingesetzt ist, wobei die Hülse (120) an der in der radialen Richtung äußeren Seite der Röhre (110) angeordnet ist und der erste gefaltete Abschnitt (120a) und der zweite gefaltete Abschnitt (120b) in Verbindung mit dem Abdichtungselement (210B, 210C) sind.

4. Hydraulikaktor (10) nach Anspruch 3, wobei:
das Abdichtungselement (210B) einen zweiten Abschnitt (216) mit kleinem Durchmesser umfasst, der einen Außendurchmesser aufweist, der kleiner ist als der Außendurchmesser des Körperabschnitts (211B),
der zweite Arretierungsring (270) an einer in der radialen Richtung äußeren Seite des zweiten Abschnitts (216) mit kleinem Durchmesser angeordnet ist und
wenigstens ein Innendurchmesser des zweiten Arretierungsrings (270) kleiner ist als der Außendurchmesser des Körperabschnitt (211B).

5. Hydraulikaktor (10) nach Anspruch 1, wobei der erste gefaltete Abschnitt (120a) an die Hülse (120) geklebt ist, die an einer in der radialen Richtung äußeren Seite der Röhre (110) angeordnet ist.

6. Hydraulikaktor (10) nach Anspruch 1, wobei ein elastisches Element, das eine bahnartige Form aufweist, zwischen der Hülse (120), die an einer in der radialen Richtung äußeren Seite der Röhre (110) angeordnet ist und dem ersten gefalteten Abschnitt (120a) angeordnet ist.

7. Hydraulikaktor (10) nach Anspruch 1, wobei ein elastisches Element (250, 250A), das eine bahnartige Form aufweist, zwischen dem ersten gefalteten Abschnitt (120a) und dem Kröpfungselement (230, 230A, 230B, 230C) angeordnet ist.

8. Hydraulikaktor (10) nach Anspruch 3, wobei ein elastisches Element, das eine bahnartige Form aufweist, zwischen dem ersten gefalteten Abschnitt (120a) und dem zweiten gefalteten Abschnitt (120b) angeordnet ist.

9. Hydraulikaktor (10) nach Anspruch 1, wobei:
der Kord (121) aus einer organischen Faser geformt ist,
der Hydraulikaktor (10) eine Überzugsschicht (122, 123) umfasst, die eine Oberfläche des Kords (121) überzieht, und
die Überzugsschicht (122, 123) aus einem Gemisch von warmaushärtendem Harz und Latex geformt ist.

10. Hydraulikaktor (10) nach Anspruch 9, wobei die Überzugsschicht (122, 123) durch Verwendung einer wässrigen Gemischlösung geformt ist, in der eine feste Fraktion (Gew.-%) des warmaushärtenden Harzes und des Latex in einem Bereich zwischen 15 % und 50 % liegt.

11. Hydraulikaktor (10) nach Anspruch 9 oder 10, wobei das warmaushärtende Harz aus Phenolharz, Resorcinharz, Urethanharz oder einem Gemisch von verschiedenen Harzen davon geformt ist.

12. Hydraulikaktor (10) nach einem der Ansprüche 9 bis 11, wobei das Latex aus VP-Latex, SBR-Latex, NBR-Latex oder einem Gemisch von verschiedenen Latizes davon geformt ist.

13. Hydraulikaktor (10) nach einem der Ansprüche 9 bis 12, wobei der Abdichtungsmechanismus eine Gummibahn umfasst, die mit der Hülse (120) in Berührung gebracht wird.

14. Hydraulikaktor (10) nach Anspruch 1, wobei:
der Kord (121) aus Stahl geformt ist,
der Hydraulikaktor (10) eine Überzugsschicht (122, 123) umfasst, die eine Oberfläche des Kords (121) überzieht, und
die Überzugsschicht (122, 123) aus thermoplastischem Harz, warmaushärtendem Harz oder einem Gemisch aus dem thermoplastischem Harz und dem warmaushärtenden Harz geformt ist.

15. Hydraulikaktor (10) nach Anspruch 14, wobei die Überzugsschicht (122, 123) aus olefinbasiertem thermoplastischem Elastomer (TPE), polyesterbasiertem thermoplastischem Elastomer (TPE), Polypropylen (PP), Polyethylen (PE), Polyvinylchlorid (PVC), Polyamid (PA), Phenolharz, Epoxidharz oder Urethanharz oder einem Gemisch von verschiedenen Harzen davon geformt ist.

## Revendications

1. Actionneur hydraulique (10) comprenant :
une partie principale d'actionneur (100) formée par un tube (110) présentant une forme cylindrique qui est dilaté et contracté par la pression d'un fluide, et un manchon (120) formé comme un corps structurel dans lequel des câbles (121) orientés dans une direction prédéterminée sont tressés, le manchon (120) étant configuré afin de couvrir une périphérie extérieure du tube (110) ; et
un mécanisme d'étanchéité (200, 200A, 200B, 200C) qui scelle une partie d'extrémité de la partie principale d'actionneur (100) dans une direction axiale,
dans lequel :
le mécanisme d'étanchéité (200, 200A, 200B, 200C) comprend :
un élément d'étanchéité (210, 210A, 210B, 210C) présentant une partie de corps (211, 211A, 211B, 211C) et une partie de bride (212, 212A) ;
un élément de calfeutrage (230, 230A, 230B, 230C) qui calfeutre la partie principale de l'actionneur (100) en combinaison avec l'élément d'étanchéité (210, 210A, 210B, 210C) ; et
un premier élément de verrouillage (220) qui verrouille le manchon (120) ;
la partie de corps (211, 211A, 211B, 211C) est insérée dans le tube (110) ;
la partie de bride (212, 212A) se poursuit jusqu'à la partie de corps (211, 211A, 211B, 211C) et un diamètre extérieur de la partie de bride (212, 212A) le long d'une direction radiale de la partie principale d'actionneur (100) est supérieur à un diamètre extérieur (R3) de la partie de corps (211, 211A, 211B, 211C) ;
le premier élément de verrouillage (220) verrouille le manchon (120) sur un côté extérieur de la partie de corps (211, 211A, 211B, 211C) dans la direction radiale ;
**caractérisé en ce que**
le premier élément de verrouillage (220) est une première bague de verrouillage (220) et
le manchon (120) comprend une première partie repliée (120a), repliée par le biais de la première bague de verrouillage (220) ; et
l'élément de calfeutrage (230, 230A, 230B, 230C) calfeutre le tube (110) dans lequel la partie de corps (211, 211A, 211B, 211C) est insérée, le manchon (120) étant situé sur le côté extérieur du tube (110) dans la direction radiale, et la première partie repliée (120a) en combinaison avec l'élément d'étanchéité (210, 210A, 210B, 210C).

2. Actionneur hydraulique (10) selon la revendication 1, dans lequel :
l'élément d'étanchéité (210, 210B) comprend une première partie à petit diamètre (214) présentant un diamètre externe inférieur au diamètre externe de la partie de corps (211, 211B) ;
la première bague de verrouillage (220) est agencée sur un côté extérieur de la première partie de petit diamètre (214) dans la direction radiale ; et
au moins un diamètre interne (R1) de la première bague de verrouillage (220) est inférieur au diamètre externe (R3) de la partie de corps (211, 211B).

3. Actionneur hydraulique (10) selon la revendication 1, dans lequel :
le mécanisme d'étanchéité (200B, 200C) comprend une seconde bague de verrouillage (270, 270C) qui verrouille le manchon (120) sur le côté extérieur de la partie de corps (211B, 211C) dans la direction radiale et dans une position plus proche d'un centre de la partie principale d'actionneur (100) dans la direction axiale que la première bague de verrouillage (220) ;
le manchon (120) comprend une seconde partie repliée (120b) repliée par le biais de la seconde bague de verrouillage (270, 270C) ;
la seconde partie repliée (120b) se poursuit jusqu'à la première partie repliée (120a) ; et
l'élément de calfeutrage (230, 230A, 230B, 230C) calfeutre la partie de corps (211B, 211C) insérée dans le tube (110), le manchon (120) étant situé sur le côté extérieur du tube (110) dans la direction radiale, la première partie repliée (120a) et la seconde partie repliée (120b), en combinaison avec l'élément d'étanchéité (210B, 210C).

4. Actionneur hydraulique (10) selon la revendication 3, dans lequel :
l'élément d'étanchéité (210B) comprend une seconde partie de petit diamètre (216) présentant un diamètre externe inférieur au diamètre externe de la partie de corps (211B) ;
la seconde bague de verrouillage (270) est située sur un côté extérieur de la seconde partie de petit diamètre (216) dans la direction radiale ; et
au moins un diamètre interne de la seconde bague de verrouillage (270) est inférieur au diamètre externe de la partie de corps (211B).

5. Actionneur hydraulique (10) selon la revendication 1, dans lequel la première partie repliée (120a) adhère au manchon (120) situé sur un côté extérieur du tube (110) dans la direction radiale.

6. Actionneur hydraulique (10) selon la revendication 1, dans lequel un élément élastique présentant une forme de feuille est agencé entre le manchon (120) situé sur un côté extérieur du tube (110) dans la direction radiale et la première partie repliée (120a).

7. Actionneur hydraulique (10) selon la revendication 1, dans lequel un élément élastique (250, 250A) présentant une forme de feuille est agencé entre la première partie repliée (120a) et l'élément de sertissage (230, 230A, 230B, 230C).

8. Actionneur hydraulique (10) selon la revendication 3, dans lequel un élément élastique présentant une forme de feuille est agencé entre la première partie repliée (120a) et la seconde partie repliée (120b).

9. Actionneur hydraulique (10) selon la revendication 1, dans lequel :
le câble (121) est formé de fibres organiques ;
l'actionneur hydraulique (10) comprend une couche de revêtement (122, 123) qui recouvre une surface du câble (121) ; et
la couche de revêtement (122, 123) est formée d'un mélange de résine thermodurcissable et de latex.

10. Actionneur hydraulique (10) selon la revendication 9, dans lequel la couche de revêtement (122, 123) est formée en utilisant une solution de mélange aqueux dans laquelle une fraction solide (% en poids) de la résine thermodurcissable et du latex est dans une plage comprise entre 15 % et 50 %.

11. Actionneur hydraulique (10) selon la revendication 9 ou 10, dans lequel la résine thermodurcissable est formée de résine de phénol, de résine de résorcinol, de résine d'uréthane, ou d'un mélange de plusieurs résines de celles-ci.

12. Actionneur hydraulique (10) selon l'une quelconque des revendications 9 à 11, dans lequel le latex est formé de latex VP, de latex SBR, de latex NBR, ou d'un mélange de plusieurs latex de ceux-ci.

13. Actionneur hydraulique (10) selon l'une quelconque des revendications 9 à 12, dans lequel le mécanisme d'étanchéité comprend une feuille de caoutchouc mise en contact avec le manchon (120).

14. Actionneur hydraulique (10) selon la revendication 1, dans lequel :
le câble (121) est constitué d'acier ;
l'actionneur hydraulique (10) comprend une couche de revêtement (122, 123) qui recouvre une surface du câble (121) ; et
la couche de revêtement (122, 123) est formée de résine thermoplastique, de résine thermodurcissable, ou d'un mélange de la résine thermoplastique et de la résine thermodurcissable.

15. Actionneur hydraulique (10) selon la revendication 14, dans lequel la couche de revêtement (122, 123) est formée d'un élastomère thermoplastique à base d'oléfine (TPE), d'un élastomère thermoplastique à base de polyester (TPE), de polypropylène (PP), de polyéthylène (PE), de chlorure de polyvinyle (PVC), de polyamide (PA), de résine de phénol, de résine époxy, ou de résine d'uréthane, ou d'un mélange de plusieurs résines de celles-ci.
